# EUROPÄISCHE PATENTANMELDUNG

(11) **EP 0 823 240 A1**
(43) Veröffentlichungstag der Anmeldung: **11.02.1998**
(21) Anmeldenummer: 97113529.8
(22) Anmeldetag: 06.08.1997
(51) Int. Cl.: A61B 17/04

(54) **Fadensicherung für chirurgisches Nahtmaterial**

(30) Priorität: 08.08.1996 DE 29613728 U
(71) Anmelder: Endotec Vertriebs- und Beratungsgesellschaft für Medizintechnik mbH, 51399 Burscheid (DE)
(72) Erfinder: Kluger Patrik, 89155 Erbach. (DE)
(74) Vertreter: Selting, Günther, Dipl.-Ing.

(57) **Zusammenfassung**

Eine Fadensicherung für chirurgisches Nahtmaterial weist ein Hülsenteil (20) und ein in das Hülsenteil (20) in Längsrichtung einsteckbares Klemmteil (21) auf. Das durch das Hülsenteil (20) hindurchgeführte Nahtmaterial (12c) ist zwischen Hülseninnenseite und Klemmteilaußenseite von dem eingsteckten Klemmteil (21) festklemmbar.

## Beschreibung

Die Erfindung bezieht sich auf eine Fadensicherung für chirurgisches Nahtmaterial.

Zur Refixation von von Knochen abgerissenen oder abgelösten Weichteilen, wie Muskeln, Kapseln und Bindern, werden verschiedene Befestigungsmittel eingesetzt, um die abgerissenen Weichteile am Ablöseort zu fixieren und ein Anwachsen der Weichteile zu ermöglichen. Bei der Refixation der Weichteile mit Hilfe von Schrauben oder Dübeln kann der Knochen wegen der Größe der Befestigungsmittel und der auftretenden Spannungen geschädigt werden. Beim Einsatz derartig großer Befestigungsmittel können außerdem abstoßende Reaktionen des umliegenden Gewebes auftreten, selbst wenn es sich um Mittel aus resorbierbarem Material handelt.

Bei der Verwendung von chirurgischem Nahtmaterial, das biodegradierbar ist, ist die Verträglichkeit höher. Zur Fixierung des Nahtmaterials müssen häufig Knoten zum Sichern bzw. Sperren des Nahtmaterials vor einer Bohrungsöffnung geknotet werden. Das Knoten des Nahtmaterials in vivo erfordert große Geschicklichkeit und macht die Anwendung transorthoskopischer Verfahren erforderlich, bei dem der Chirurg mit Hilfe eines Endoskops das Knoten des Nahtmaterials optisch verfolgen kann. Dieses Verfahren ist daher sehr umständlich und aufwendig.

Es ist Aufgabe der Erfindung, eine einfache Fadensicherung für chirurgisches Nahtmaterial zu schaffen.

Die Aufgabe wird erfindungsgemäß mit den Merkmalen des Anspruchs 1 gelöst.

Die erfindungsgemäße Fadensicherung weist ein Hülsenteil und ein in das Hülsenteil in Längsrichtung einsteckbares Klemmteil auf. Das durch das Hülsenteil hin-durchgeführte Nahtmaterial ist zwischen der Hülseninnenseite und der Klemmteilaußenseite des eingesteckten Klemmteils festklemmbar. Damit ist eine einfache Möglichkeit geschaffen, an einem Faden aus chirurgischem Nahtmaterial eine Fadensicherung zu applizieren. Dazu wird das zu sichernde Ende des Fadens durch das Hülsenteil gezogen und festgezogen. In dieser Fixationsstellung des Fadens in der Hülse wird ein entsprechendes Klemmteil in das Hülsenteil eingesteckt, wodurch der Faden zwischen der Hülseninnenseite und der Klemmteilaußenseite unlösbar festgeklemmt wird. Die Sicherung des Fadens ist durch einen sehr einfachen Handgriff vornehmbar, so daß in der Regel keine transorthoskopischen Verfahren angewendet werden müssen. Die Fadensicherung ist sehr klein, so daß die Gefahr von Abstoßungsreaktionen des umliegenden Gewebes gering ist. Der Vorgang der Fadensicherung chirurgischen Nahtmaterials wird beschleunigt, vereinfacht und ist mit einfachen Hilfsmitteln vornehmbar.

Das Klemmteil weist vorzugsweise an einem dem Einsteckende gegenüberliegenden Ende einen radialen Kragen auf. Dieser Kragen hat zum einen die Funktion, die Einstecktiefe des Klemmteils in das Hülsenteil zu begrenzen und genau zu definieren. Dadurch wird eine definierte Klemmwirkung und -kraft erreicht, so daß der Faden nicht zu locker gesichert oder aber gequetscht, geknickt oder abgeschert wird. Zum anderen bewirkt der Kragen ein Umlegen des aus der Hülse heraustretenden Fadens aus der axialen Orientierung in eine radiale Orientierung. Dadurch wird der Faden in die Öffnungskante des Hülsenteils gedrückt, leicht eingekerbt und dadurch unverrutschbar an der Hülsenöffnungskante verankert.

Der Kragen kann eine in Längsrichtung durchgehende Ausnehmung aufweisen, die tiefer als der Querschnitt des Fadens ist und zur axialen Durchführung des Fadens dient. Der Faden wird definiert durch diese Ausnehmung in axialer Richtung geführt und tritt in axialer Richtung aus dem Kragen des Klemmteils heraus.

Das Hülsenteil ist vorzugsweise als Zylinderkörper und das Klemmteil als Konuskörper ausgebildet. Bei dieser Kombination kann das zum Einsteckende hin zulaufende konusförmige Klemmteil einfach in das zylinderförmige Hülsenteil eingesteckt werden. Je tiefer das Hülsenteil in den Zylinderkörper eingeschoben wird, desto mehr wird es zentriert und der Faden zwischen Zylinderinnenwand und Konuskörperaußenwand eingeklemmt. Dadurch wird ein dosierbares Festklemmen des Nahtmaterials in der Fadensicherung ermöglicht und ein zu lockeres oder zu festes Verklemmen des Nahtmaterials aufgrund von unterschiedlichen Fadendurchmessern ist durch Variation der Einstecktiefe korrigierbar.

Der Konuskörper kann eine sich über mindestens ein Teil seiner Länge erstreckenden Längsschlitz zur Aufnahme eines Teils des Querschnitts des Nahtmaterials aufweisen. Beim Verklemmen des Klemmteils in dem Hülsenteil wird das Nahtmaterial in diesem Längsschlitz in Längsrichtung geführt. Nur ein Teil des Querschnitts des Nahtmaterials ragt aus der Kontur des Konuskörpers hervor. Nur dieser Teil wird bei vollständig eingestecktem Klemmteil zwischen Hülsenteil und Klemmteil verklemmt bzw. eingekerbt. Dadurch wird verhindert, daß das Nahtmaterial unerwünschterweise zu weit eingekerbt oder gar abgeschert wird. Die Längsschlitze des Konuskörpers und des Kragens liegen vorzugsweise in einer axialen Linie hintereinander.

Das Klemmteil weist vorzugsweise eine axiale Haltebohrung an dem dem Einsteckende gegenüberliegenden Ende auf. Die Haltebohrung ermöglicht die Aufnahme des Klemmteils auf ein entsprechendes Applikationswerkzeug, wodurch das Führen und Einstecken des Klemmteils in das Hülsenteil erleichtert wird.

Das Hülsenteil und das Klemmteil sind vorzugsweise bioresorbierbar oder biodegradierbar ausgebildet. Die Fadensicherung wird im Körper des Patienten zersetzt oder abgebaut. Nach dem Anwachsen der fixierten Weichteile an den Knochen ist kein weiterer Eingriff zur Entfernung der Fadensicherung erforderlich.

Zur Installation der Fadensicherung in einem Patientenkörper kann ein Applikator vorgesehen werden, der ein Stützrohr aufweist, in dem ein stabförmiger Klemmteilhalter in Längsrichtung verschiebbar steckt. Das Klemmteil ist in dem Stützrohr aufnehmbar und kann gegen das Hülsenteil drücken. Zum Halten des Klemmteils weist der Klemmteilhalter an seiner Stirnseite eine Haltevorrichtung auf. Nach dem Einfädeln des Hülsenteils auf dem Faden wird der Faden ebenfalls durch das Stützrohr gezogen. In dem Stützrohr steckt der Klemmteilhalter, an dessen dem Hülsenteil zugewandten Ende das Klemmteil steckt. Mit dem Stützrohr wird das Hülsenteil auf den Ort, wo der Faden aus dem Gewebe heraustritt, gedrückt und der Faden gleichzeitig festgezogen. Wenn die gewünschte Fadenspannung eingestellt ist, wird mit dem Klemmteilhalter das Klemmteil auf das offene Ende des Hülsenteils gesteckt und eingeschoben, bis der Faden zwischen Hülsenteil und Klemmteil festgeklemmt ist. Der Klemmteilhalter wird nun von dem Klemmteil abgezogen und zusammen mit dem Stützrohr aus dem Patientenkörper herausgezogen. Damit ist ein einfaches Instrument geschaffen, das die Applikation der erfindungsgemäßen Fadensicherung innerhalb eines Patientenkörpers erlaubt, ohne daß dazu orthoskopische Verfahren erforderlich sind. Der Applikator gewährleistet eine definierte und feste Sicherung des Fadens.

Vorzugsweise weist die Haltevorrichtung des Applikators einen Ansatz zum Halten und Zentrieren des Klemmteils an der Haltebohrung des Klemmteils auf. Damit ist eine einfache und zuverlässige sowie leicht lösbare Haltevorrichtung für das Klemmteil an dem Klemmteilhalter realisiert.

Der Klemmteilhalter weist vorzugsweise eine durchgehende Längsnut auf, die tiefer als der Querschnitt des Nahtmaterials ist. In dieser Längsnut sind ein oder mehrere Fäden des Nahtmaterials führbar, ohne daß sie aus dem Klemmteilhalter hervorragen. Das Nahtmaterial wird dadurch widerstandsfrei in dem Klemmteilhalter geführt, so daß die Fadenspannung so lange von außen frei einstellbar ist, wie das Klemmteil noch nicht in dem Hülsenteil steckt.

Der Applikator kann zunächst in Ruhe vorbereitet und der Faden eingefädelt werden. Anschließend wird das Hülsenteil auf die Fadenaustrittsstelle aufgesetzt und die gewünschte Fadenspannung durch Ziehen an dem aus dem freien Stützrohrende heraustretenden Faden eingestellt.

Im folgenden wird unter Bezugnahme auf die Zeichnungen ein Ausführungsbeispiel der Erfindung näher erläutert.

Es zeigen:
- Abbildung 1: eine Fadensicherung, die mit Hilfe eines Applikators an einer Schultergelenkpfanne installiert wird,
- Abbildung 2: die Fadensicherung der Fig. 1 im installierten Zustand,
- Fig. 3: eine Schnittdarstellung des Applikators, des Hülsenteils und des Klemmteils vor der Installation, und
- Fig. 4: eine Schnittdarstellung des Applikators und der Fadensicherung der Fig. 3 im installierten Zustand.

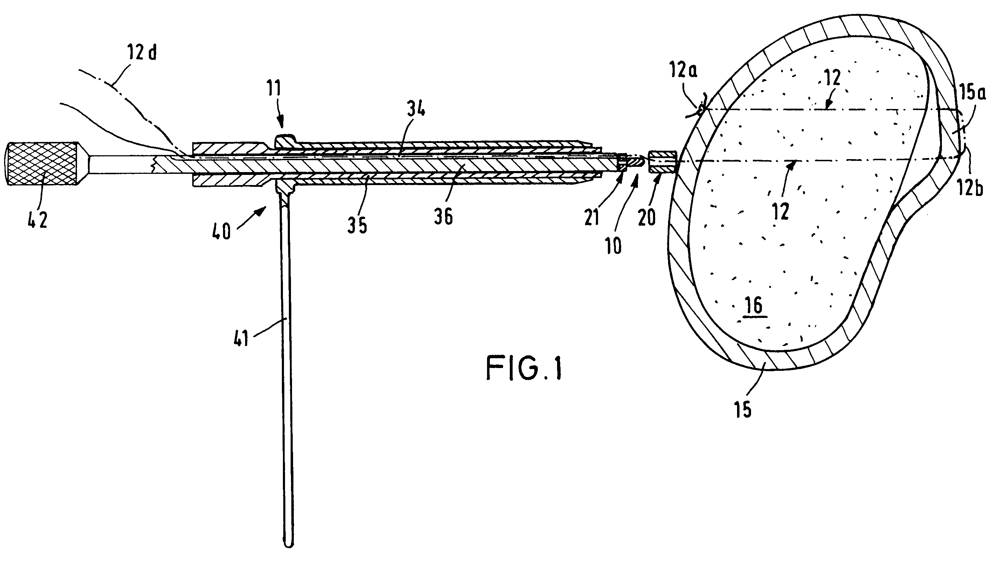

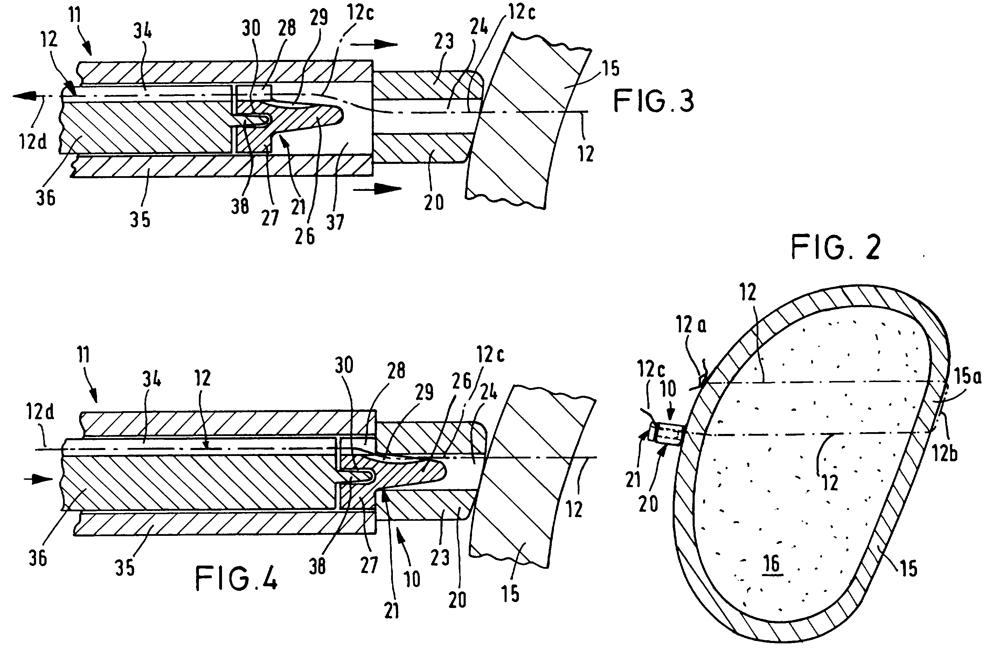

In den Figuren 1 und 2 ist eine Fadensicherung 10 dargestellt, die mit Hilfe eines Applikators 11 zum Sichern eines Fadenendes 12c eines Fadens 12 aus chirurgischem Nahtmaterial installiert wird. Mit dem Faden 12 wird ein abgelöster Teil 15a eines Labrum glenoidale 15 einer Schulterpfanne 16 fixiert. Dadurch wird ein mehrwöchiges Anwachsen des abgelösten Teils 15a ermöglicht und die Verletzung dadurch ausgeheilt. Die Fadensicherung 10 sichert das Fadenende 12c am Austrittsort des Fadens 12 aus der Gelenkpfanne 16.

Wie die Fign. 3 und 4 zeigen, weist die Fadensicherung 10 ein zylindrisches Hülsenteil 20 auf, in das ein Klemmteil 21 eingesteckt ist. Das Hülsenteil 20 besteht aus einem Zylinderkörper 23 mit einer durchgehenden axialen Bohrung 24. Das Klemmteil 21 weist einen Konuskörper 26 und einen am breiten Ende des Konuskörpers 26 angeordneten Kragenkopf 27 auf. Der Konuskörper 26 läuft unter einem spitzen Winkel zu, wobei die Konusspitze abgerundet ist. Der Kragenkopf 27 weist am Kragenrand als eine Ausnehmung eine durchgehende Längsnut 28 auf, die tiefer und breiter als der Querschnitt des Fadens 12 ist. Dadurch kann der Faden 12 in der Längsnut 28 widerstandsfrei geführt werden, ohne daß der Faden 12 aus dem Kragenkopf 27 seitlich hervorsteht.

Der Konuskörper 26 weist ebenfalls eine Längsnut 28 auf, die sich längenmäßig ungefähr von der Konuskörpermitte bis hinein in den Kragenkopfansatz erstreckt. Die Tiefe und Breite dieser Längsnut 29 ist geringer als der Querschnitt des Fadens 12. Dadurch ragt der in die Längsnut 29 eingelegte Faden 12 stets mit einem Teil seines Querschnitts aus der Längsnut 29 heraus. Die Längsnut 29 des Konuskörpers 26 und die Längsnut 28 in dem Kragenkopf 27 sind axial in einer Linie hintereinander angeordnet, so daß der Faden 12 in gerader Linie in die Nuten 28,29 eingelegt werden kann. Der Kragenkopf 27 weist an dem dem Konuskörper 26 abgewandten Ende eine axiale Haltebohrung 30 auf, die als Sackbohrung ausgebildet ist.

Der Applikator 11 besteht aus einem zylindrischen Stützrohr 35, in dem kolbenartig ein stabförmiger Klemmteilhalter 36 verschiebbar gelagert ist. Der Innendurchmesser der Stützrohrbohrung 37 ist geringfügig größer als der Außendurchmesser des Kragenkopfes 27 des Klemmteils 21, wie auch des Klemmteilhalters 36. Das Klemmteil 21 und der Klemmteilhalter 36 können daher widerstandsarm in dem Stützrohr 35 in Längsrichtung bewegt werden. Der Klemmteilhalter 36 weist einen zentralen axialen Ansatz 38 auf, der genau in die axiale Haltebohrung 30 paßt. Der Ansatz 38 hält mit geringer Klemmkraft das Klemmteil 21 an dem Klemmteilhalter 36 fest.

Der Klemmteilhalter 36 weist eine über die gesamte Länge durchgehende Längsnut 34 auf, die tiefer und breiter als der Querschnitt des Nahtmaterialfadens 12 ist, so daß der Faden 12 widerstandsfrei in der Längsnut 34 bewegbar ist.

Wie in Fig. 1 gezeigt, weist der Applikator 11 eine zylindrische Haltevorrichtung 40 auf, in die das Stützrohr 35 eingeschoben ist. Die Haltevorrichtung 40 weist einen radial abstehenden Griffkörper 41 zum bequemen Halten des Applikators 11 auf.

In den Fign. 1 bis 4 ist beispielhaft die Refixierung des abgelösten Teils 15a des Labrum glenoidale 15 an der Schulterpfanne 16 dargestellt. Zunächst wird der an einem freien Ende 12a mit einem Stoppknoten verknotete Nahtmaterialfaden 12 U-förmig durch zwei Bohrungen in der Gelenkpfanne 16 hindurchgezogen, so daß ein bogenförmiger Teil 12b des Fadens 12 den abgelösten Gewebeteil 15a zweimal durchstößt und bei angezogenem Faden 12 an den Rand der Gelenkpfanne 16 anzieht (Fig. 2).

Anschließend wird auf das verbleibende freie Ende 12d des Fadens 12 das Hülsenteil 20 aufgezogen und zum Austrittsort des Fadens 12 aus der Gelenkpfanne 16 geschoben. Anschließend wird der Faden 12 durch das Stützrohr 35 und die ihn haltende Haltevorrichtung 40 gezogen. Schließlich wird der Klemmteilhalter 36 in das Stützrohr eingesteckt, wobei der Faden 12 in der Längsnut 34 geführt wird, so daß der Faden 12 nicht zwischen dem Klemmteilhalter 36 und dem Stützrohr 35 eingeklemmt wird. Der Klemmteilhalter 36 wird eingeschoben, bis ein Anschlagkörper 42 am rückwärtigen Ende des Klemmteilhalters 36 die axiale Bewegung des Klemmteilhalters 36 begrenzt und das den Ansatz 38 aufweisende Ende aus dem anderen Ende des Stützrohrs 35 wieder herausgetreten ist. Nun wird das Klemmteil 21 mit seiner Haltebohrung 30 auf den Ansatz 38 des Klemmteilhalters 36 aufgesetzt, wobei die Kragennut 28 des Klemmteils 21 mit der Längsnut 34 des Klemmteilhalters 36 in einer Linie fluchten muß. Der Faden 12 wird dann in den Längsnuten 28,29 und 34 geführt. Die Applikation der Fadensicherung 10 erfolgt, indem das Stützrohr 35 axial auf den Zylinderkörper 23 aufgesetzt und der Zylinderkörper 23 gegen die Gelenkpfanne 16 gedrückt wird (Fig. 3). Bei gleichzeitigem Ziehen des Fadens 12 am Fadenende 12d mit ungefähr 2 kg wird das Klemmteil 21 mit dem Klemmteilhalter 36 in den Zylinderkörper 23 eingesteckt. Dabei verklemmt der aus der Gelenkpfanne 16 austretende Fadenabschnitt 12c zwischen der Öffnungskante des Zylinderkörpers 23 und dem Nutansatz der Nut 29 des Konuskörpers 26. Der Fadenabschnitt 12c liegt dabei querschnittsmäßig teilweise in der Längsnut 29 des Konuskörpers 26 und ragt nur teilweise aus der Längsnut 29 heraus. Dieser überstehende Querschnittsteil des Fadenabschnitts 12c wird von dem Öffnungsrand des Zylinderkörpers 23 leicht eingekerbt, wodurch, neben der Haftreibung zwischen dem Fadenabschnitt 12c einerseits und dem Zylinderkörper 23 und Konuskörper 26 andererseits der Faden 12c auch durch die Kerbklemmung gegen Lösen gesichert wird. Anschließend kann der Applikator 11 von dem Hülsenteil 20 und dem Klemmteil 21 abgezogen und entfernt werden. Nachdem der überstehende Fadenabschnitt abgeschnitten wurde, kann die Wunde wieder verschlossen werden.

Das Hülsenteil 20 und das Klemmteil 21 sowie das Nahtmaterial 12 bestehen aus biodegradierbarem Material, so daß sie nach wenigen Monaten vollständig zersetzt sind. Alle Teile des Applikators 12 sind aus rostfreiem Stahl hergestellt, können ggf. aber auch aus anderen Materialien, beispielsweise Kunststoff gefertigt sein.

Die Länge des Zylinderkörpers beträgt 5 mm, sein Außendurchmesser 4 mm und sein Innendurchmesser 2 mm. Das Klemmteil 21 hat ebenfalls eine Länge von 5 mm, die Tiefe der Haltebohrung beträgt 3 mm. Der Kragendurchmesser ist ungefähr gleich dem Außendurchmesser des Hülsenteils 20. Die Tiefe der Kragennut 28 beträgt ungefähr 1 mm, die Tiefe der Längsnut 29 des Konuskörpers 26 ist dagegen geringer.

## Patentansprüche

1. Fadensicherung für chirurgisches Nahtmaterial (12), mit einem Hülsenteil (20) und einem in das Hülsenteil (20) in Längsrichtung einsteckbaren Klemmteil (21),
wobei das durch das Hülsenteil (20) hindurchgeführte Nahtmaterial (12c) zwischen Hülseninnenseite und Klemmteilaußenseite von dem eingesteckten Klemmteil (21) festklemmbar ist.

2. Fadensicherung nach Anspruch 1, dadurch gekennzeichnet, daß das Klemmteil (21) an einem dem Einsteckende gegenüberliegenden Ende einen radialen Kragen (27) aufweist.

3. Fadensicherung nach Anspruch 2, dadurch gekennzeichnet, daß der Kragen (27) eine in Längsrichtung durchgehende Ausnehmung (28) tiefer als der Querschnitt des Nahtmaterials (12) zur axialen Durchführung des Nahtmaterials (12) aufweist.

4. Fadensicherung nach einem der Ansprüche 1 bis 3, dadurch gekennzeichnet, daß das Hülsenteil (20) als Zylinderkörper (23) ausgebildet ist und das Klemmteil (21) als Konuskörper (26) ausgebildet ist.

5. Fadensicherung nach Anspruch 4, dadurch gekennzeichnet, daß der Konuskörper (26) einen sich über mindestens einen Teil der Länge des Konuskörpers (26) erstreckenden Längsschlitz (29) zur Aufnahme eines Teils des Querschnitts des Nahtmaterials (12) aufweist.

6. Fadensicherung nach einem der Ansprüche 1 bis 5, dadurch gekennzeichnet, daß das Klemmteil (21) eine axiale Haltebohrung (30) an dem dem Einsteckende gegenüberliegende Ende aufweist.

7. Fadensicherung nach einem der Ansprüche 1 bis 6, dadurch gekennzeichnet, daß das Hülsenteil (20) und das Klemmteil (21) bioresorbierbar oder biodegradierbar sind.

8. Applikator zur Installation der Fadensicherung (10) in einem Körper nach einem der Ansprüche 1 bis 7, mit einem Stützrohr (35), in dem ein stabförmiger Klemmteilhalter (36) in Längsrichtung verschiebbar steckt, wobei das Klemmteil (21) in dem Stützrohr (35) aufnehmbar ist und gegen das Hülsenteil (20) drücken kann, und der Klemmteilhalter (36) an einer Stirnseite eine Haltevorrichtung für das Klemmteil (21) aufweist.

9. Applikator nach Anspruch 8, dadurch gekennzeichnet, daß die Haltevorrichtung (40) einen Ansatz (38) zum Halten und Zentrieren des Klemmteils (21) an der Haltebohrung (30) des Klemmteils (21) aufweist.

10. Applikator nach Anspruch 8 oder 9, dadurch gekennzeichnet, daß der Klemmteilhalter (36) eine durchgehende Längsnut (34) tiefer als der Querschnitt des Nahtmaterials (12) aufweist.
